# EUROPEAN PATENT SPECIFICATION

(11) **EP 1 831 402 B1**
(45) Date of publication and mention of the grant of the patent: **09.06.2010**
(21) Application number: 05855433.8
(22) Date of filing: 21.12.2005
(51) Int. Cl.: C12Q 1/68

(54) **GENETIC VARIANTS OF VKORC1 PREDICTING WARFARIN SENSITIVITY**
WARFARINSENSIBILITÄT VORHERSAGENDE GENETISCHE VARIANTEN VON VKORC1
VARIANT GENETIQUE PREVOYANT LA SENSIBILITE A LA WARFARINE

(30) Priority: 21.12.2004 US 638837 P; 10.05.2005 US 679694 P
(43) Date of publication of application: 12.09.2007
(73) Proprietor: Academia Sinica, Nan-Kang, Taipei (TW)
(72) Inventor: CHEN, Yuan-Tsong No. 9 Alley I Lane 61, Taipei City, 000115 (TW); YUAN, Hsiang-yu, Taipei, 000115 (TW); CHEN, Jin-jer, Taipei (TW)
(74) Representative: Brookes Batchellor LLP
(86) International application number: PCT/US2005/046869
(87) International publication number: WO 2006/069339

(56) References cited:
- WO-A-2006/044686
- WO-A2-2005/030039
- ROST S ET AL: "Mutations in VKORC1 cause warfarin resistance and multiple coagulation factor deficiency type 2" NATURE, NATURE PUBLISHING GROUP, LONDON, GB, vol. 427, no. 6974, 5 February 2004 (2004-02-05), pages 537-541, XP002318816 ISSN: 0028-0836
- DATABASE EMBL [Online] 11 April 2004 (2004-04-11), "Homo sapiens vitamin K epoxide reductase complex, subunit 1 (VKORC1) gene, complete cds." XP002391711 retrieved from EBI accession no. EM_PRO:AY587020 Database accession no. AY587020

## Description

### FIELD OF INVENTION

This invention relates to methods of dosing drugs, particularly warfarin.

### REFERENCES

1. Hirsh, J. (1992) Antithrombotic therapy in deep vein thrombosis and pulmonary embolism. Am. Heart J., 123, 1115-1122.
2. Laupacis, A., Albers, G., Dalen, J., Dunn, M., Feinberg, W. and Jacobson, A. (1995) Antithrombotic therapy in atrial fibrillation. Chest, 108, 352S-359S.
3. Stein, P.D., Alpert, J.S., Copeland, J., Dalen, J.E., Goldman, S. and Turpie, A.G. (1995) Antithrombotic therapy in patients with mechanical and biological prosthetic heart valves. Chest, 108, 371S-379S.
4. Hirsh, J., Dalen, J., Anderson, D.R., Poller, L., Bussey, H., Ansell, J. and Deykin, D. (2001) Oral anticoagulants: mechanism of action, clinical effectiveness, and optimal therapeutic range. Chest, 119, 8S-21 S.
5. Loebstein, R., Yonath, H., Peleg, D., Almog, S., Rotenberg, M., Lubetsky, A., Roitelman, J., Harats, D., Halkin, H. and Ezra, D. (2001) Interindividual variability in sensitivity to warfarin--Nature or nurture? Clin. Pharmacol. Ther., 70, 159-164.
6. Takahashi, H., Wilkinson, G.R., Caraco, Y., Muszkat, M., Kim, R.B., Kashima, T., Kimura, S. and Echizen, H. (2003) Population differences in S-warfarin metabolism between CYP2C9 genotype-matched Caucasian and Japanese patients. Clin. Pharmacol. Ther., 73, 253-263.
7. Zhao, F., Loke, C., Rankin, S.C., Guo, J.Y., Lee, H.S., Wu, T.S., Tan, T., Liu, T.C., Lu, W.L., Lim, Y.T. et al. (2004) Novel CYP2C9 genetic variants in Asian subjects and their influence on maintenance warfarin dose. Clin. Pharmacol. Ther., 76,210-219.
8. Yu, H.C., Chan, T.Y., Critchley, J.A. and Woo, K.S. (1996) Factors determining the maintenance dose of warfarin in Chinese patients. QJM 89, 127-135.
9. Xie, H.G., Kim, R.B., Wood, A.J. and Stein, C.M. (2001) Molecular basis of ethnic differences in drug disposition and response. Annu. Rev. Pharmacol. Toxicol., 41, 815-850. 10. Bogousslavsky, J. and Regli, F. (1985) Anticoagulant-induced intracerebral bleeding in brain ischemia. Evaluation in 200 patients with TIAs, emboli from the heart, and progressing stroke. Acta. Neurol. Scand., 71, 464-471.
11. Landefeld, C.S. and Beyth, R.J. (1993) Anticoagulant-related bleeding: clinical epidemiology, prediction, and prevention. Am. J. Med., 95, 315-328.
12. Gullov, A.L., Koefoed, B.G. and Petersen, P. (1994) Bleeding Complications to Long-Term Oral Anticoagulant Therapy. J. Thromb. Thrombolysis, 1, 17-25.
13. Kaminsky, L.S., Dunbar, D.A., Wang, P.P., Beaune, P., Larrey, D., Guengerich, F.P., Schnellmann, R.G. and Sipes, I.G. (1984) Human hepatic cytochrome P-450 composition as probed by in vitro microsomal metabolism of warfarin. Drug Metab. Dispos., 12, 470-477.
14. Rettie, A.E., Korzekwa, K.R., Kunze, K.L., Lawrence, R.F., Eddy, A.C., Aoyama, T., Gelboin, H.V., Gonzalez, F.J. and Trager, W.F. (1992) Hydroxylation of warfarin by human cDNA-expressed cytochrome P-450: a role for P-4502C9 in the etiology of (S)-warfarin-drug interactions. Chem. Res. Toxicol., 5, 54-59.
15. Kaminsky, L.S., de Morais, S.M., Faletto, M.B., Dunbar, D.A. and Goldstein, J.A. (1993) Correlation of human cytochrome P4502C substrate specificities with primary structure: warfarin as a probe. Mol. Pharmacol., 43, 234-239.
16. Xie, H.G., Prasad, H.C., Kim, R.B. and Stein, C.M. (2002) CYP2C9 allelic variants: ethnic distribution and functional significance. Adv. Drug. Deliv. Rev., 54, 1257-1270.
17. Kirchheiner, J. and Brockmoller, J. (2005) Clinical consequences of cytochrome P450 2C9 polymorphisms. Clin. Pharmacol. Ther., 77, 1-16.
18. Furuya, H., Fernandez-Salguero, P., Gregory, W., Taber, H., Steward, A., Gonzalez, F.J. and Idle, J.R. (1995) Genetic polymorphism of CYP2C9 and its effect on warfarin maintenance dose requirement in patients undergoing anticoagulation therapy. Pharmacogenetics, 5, 389-392.
19. Aithal, G.P., Day, C.P., Kesteven, P.J. and Daly, A.K. (1999) Association of polymorphisms in the cytochrome P450 CYP2C9 with warfarin dose requirement and risk of bleeding complications. Lancet, 353, 717-719.
20. Higashi, M.K., Veenstra, D.L., Kondo, L.M., Wittkowsky, A.K., Srinouanprachanh, S.L., Farin, F.M. and Rettie, A.E. (2002) Association between CYP2C9 genetic variants and anticoagulation-related outcomes during warfarin therapy. JAMA, 287, 1690-1698.
21. Nasu, K., Kubota, T. and Ishizaki, T. (1997) Genetic analysis of CYP2C9 polymorphism in a Japanese population. Pharmacogenetics, 7, 405-409.
22. Bell, R.G. and Matschiner, J.T. (1972) Warfarin and the inhibition of vitamin K activity by an oxide metabolite. Nature, 237, 32-33.
23. Wallin, R. and Martin, L.F. (1985) Vitamin K-dependent carboxylation and vitamin K metabolism in liver. Effects of warfarin. J. Clin. Invest., 76, 1879-1884.
24. Li, T., Chang, C.Y., Jin, D.Y., Lin, P.J., Khvorova, A. and Stafford, D.W. (2004) Identification of the gene for vitamin K epoxide reductase. Nature, 427,541-544.
25. Rost, S., Fregin, A., Ivaskevicius, V., Conzelmann, E., Hortnagel, K., Pelz, H.J., Lappegard, K., Seifried, E., Scharrer, I., Tuddenham, E.G. et al. (2004) Mutations in VKORC1 cause warfarin resistance and multiple coagulation factor deficiency type 2. Nature, 427, 537-541.
26. Harrington, D.J., Underwood, S., Morse, C., Shearer, M.J., Tuddenham, E.G. and Mumford, A.D. (2005) Pharmacodynamic resistance to warfarin associated with a Val66Met substitution in vitamin K epoxide reductase complex subunit 1. Thromb. Haemost., 93, 23-26.
27. D'Andrea, G., D'Ambrosio, R.L., Di Perna, P., Chetta, M., Santacroce, R., Brancaccio, V., Grandone, E. and Margaglione, M. (2005) A polymorphism in the VKORC1 gene is associated with an interindividual variability in the dose-anticoagulant effect of warfarin. Blood, 105, 645-649.
28. Massari, M.E. and Murre, C. (2000) Helix-loop-helix proteins: regulators of transcription in eucaryotic organisms. Mol. Cell Biol., 20, 429-440.
29. Terai, S., Aoki, H., Ashida, K. and Thorgeirsson, S.S. (2000) Human homologue of maid: A dominant inhibitory helix-loop-helix protein associated with liver-specific gene expression. Hepatology, 32, 357-366.
30. Bodin, L., Verstuyft, C., Tregouet, D.A., Robert, A., Dubert, L., Funck-Brentano, C., Jaillon, P., Beaune, P., Laurent-Puig, P., Becquemont, L. et al. (2005) Cytochrome P450 2C9 (CYP2C9) and vitamin K epoxide reductase (VKORC1) genotypes as determinants of acenocoumarol sensitivity. Blood, 106(1):135-140.
31. Jones, K.A., Kadonaga, J.T., Rosenfeld, P.J., Kelly, T.J. and Tjian, R. (1987) A cellular DNA-binding protein that activates eukaryotic transcription and DNA replication. Cell, 48, 79-89.
32. Rieder, M. J., A. P. Reiner, et al. (2005). Effect of VKORC1 haplotypes on transcriptional regulation and warfarin dose. N Engl J Med. 352(22): 2285-93.
33. Geisen, C., M. Watzka, et al. (2005). VKORC1 haplotypes and their impact on the inter-individual and inter-ethnical variability of oral anticoagulation. Thromb Haemost, 94(4): 773-9.
34. Veenstra, D. L., J. H. You, et al. (2005). Association of Vitamin K epoxide reductase complex 1 (VKORC1) variants with warfarin dose in a Hong Kong Chinese patient population. Pharmacogenet Genomics, 15(10): 687-91.
35. Sconce, E. A., T. I. Khan, et al. (2005). The impact of CYP2C9 and VKORC1 genetic polymorphism and patient characteristics upon warfarin dose requirements: proposal for a new dosing regimen. Blood, 106(7): 2329-33.

### BACKGROUND

Warfarin is a widely prescribed anticoagulant for the prevention of thromboembolic diseases for subjects with deep vein thrombosis, atrial fibrillation, or mechanical heart valve replacement (1-4). However, warfarin treatment is problematic because the dose requirement for warfarin is highly variable, both inter-individually and inter-ethnically (5-7). Asian populations, including the Chinese, generally require a much lower maintenance dose than Caucasians and Hispanics (6-9). Bleeding is by far the most serious complication of warfarin treatment (10-12). Much effort has been devoted to monitor the safety of this oral anticoagulant. Currently, the dose has to be closely monitored by serial determinations of blood prothrombin time using standardized international normalized ratio (INR).

Cytochrome P450, subfamily IIC, polypeptide 9 (CYP2C9) is the principal drug metabolizing enzyme that catalyzes the hydroxylation of warfarin (13-15). CYP2C9*2 and CYP2C9*3 are the polymorphisms most frequently found in the Caucasian population. Taking CYP2C9* 1 as wild type, the haplotype frequencies for CYP2C9*1/*2 and CYP2C9*1/*3 are ∼ 20% and ∼12% respectively (16, 17). The CYP2C9*2 and CYP2C9*3 variants have been shown to decrease the enzymatic activity of CYP2C9, which leads to warfarin sensitivity and, in serious cases, bleeding complications (18-20). However, both CYP2C9*2 and CYP2C9*3 are either completely absent or rare in the Asian populations (9,21). Genetic variants discovered in CYP2C9 thus far can only partially explain some of the inter-individual differences in warfarin dosage, but they cannot explain the inter-ethnic differences (6, 7,16).

Gamma-carboxylation of vitamin K-dependent clotting factors (factor II, VII, IX, and X) is essential for blood clotting. The gamma-carboxylase uses the reduced form of vitamin K and oxygen to add a carbon dioxide molecule to the side chain of glutamic acid in the clotting factors. During carboxylation, the reduced vitamin K is oxidized to vitamin K 2,3-epoxide, from which the reduced vitamin K is regenerated by vitamin K epoxide reductase for another cycle of catalysis. Warfarin blocks clotting factor synthesis by inhibiting vitamin K epoxide reductase (22, 23). Recently, the gene coding for vitamin K epoxide reductase complex, subunit 1 (VKORC1) has been cloned (24, 25) and mutations in VKORC1 gene were found in warfarin resistant patients (25, 26). An intronic polymorphism was also discovered to be associated with certain warfarin inter-individual variability in Italian patients (27).

These studies cannot explain all the warfarin dosage variability, particularly the inter-ethnic variation. Furthermore, a large part of the world's population, the, Asians, is unaccounted for. Therefore, it is desirable to find a method for predicting the proper warfarin dose range that can be more widely applied.
The gene sequence of VKORC1 including the promoter Polymorphism at position 1639 is available as EMBL Accession No. AY587020.
WO 2006/044686 discloses methods for predicting drug responses. In particular, it discloses methods for determining warfarin dosages based in the presence or absence of polymorphisms in the VKORC1 gene. WO 2006/044686 indicates that a patient carrying Homozygous clade A/A or B/B is sensitive or resistant to warfarin respectively. The clades are based on a number of haplotypes which refer to a particular combination of ten single nucleotide polymorphisms located in the VKORC1 gene.
WO2005030039 discloses a link between warfarin dosage and the SNPs at position 6853 and 9041 of SEQ ID NO: 1.

### SUMMARY

In this study, we discovered that a polymorphism in the promoter of the VKORC1 gene is associated with warfarin sensitivity. This polymorphism can explain both the inter-individual and inter-ethnic differences in warfarin dose requirements. Furthermore, the polymorphism is also associated with promoter activity. The VKORC1 promoter having a G at the -1639 position (numbered with respect to the first nucleotide of the initiation codon) is 44% more active than the promoter having an A at the same position. Patients with the -1639 A polymorphism are more sensitive to warfarin. Homozygous AA patients at the -1639 position had the lowest dose requirements, heterozygous AG had intermediate dose requirements and the homozygous GG patients had the highest dosage. Thus, the promoter sequence or activity of the VKORC1 gene of a subject can be used to predict how much warfarin should be prescribed for the subject. This method significantly improves the accuracy of warfarin dosing. Currently, patients are given an initial dose, their INR monitored periodically and warfarin dosage adjusted accordingly, until a maintenance dose that is safe for the patient can be achieved. With the present invention, the predicted dose will be much closer to the maintenance dose, thus warfarin dosing will be quicker, safer, and more economical.

Accordingly, one aspect of the present invention provides a method of predicting a dose range of a warfarin for a human patient, comprising: investigating the nucleotide at the -3673 position of SEQ ID NO: 1 of the patient; determining whether the patient is warfarin sensitive or resistant based on the nucleotide at the -3673 position wherein homozygous AA, heterozygous AG, and homozygous GG at position 3673 indicate that the patient is warfarin sensitive, intermediate sensitive and resistant, respectively; and predicting a warfarin dosage range based on the warfarin sensitivity or resistance of the patient. The more sensitive the subject is to warfarin, the higher is the risk to develop complications, such as bleeding.

In particular, the sequence at the -1639 position of the VKORC1 gene is investigated. If the nucleotide at this position is an A, the subject is more sensitive to warfarin. Thus, a subject having homozygous AA at the -1639 position of the VKORC1 gene is more sensitive than a heterozygote having an A and another nucleotide (G, C or T) at this position. In turn, the same heterozygote is more sensitive to warfarin than a subject having no A at this position.

The sequence can be investigated by assaying for an equivalent genetic marker of the -1639 A or -1639G/C/T allele, wherein the presence of the equivalent genetic marker is indicative of the presence of the corresponding allele. For example, the equivalent genetic marker may be an SNP selected from the group consisting of rs9934438, rs8050894, rs2359612 and rs7294 of the VKORC1 gene, each of which is indicative of warfarin sensitivity.

In some embodiments of the present invention, the sequence is investigated by using an oligonucleotide that specifically hybridizes with the promoter of the VKORC1 gene. Preferably, the oligonucleotide specifically hybridizes with at least 6, 8, 10, 12, 14, 16, 18, 20, 22 or 24 nucleotides spanning the -1639 position of the VKORC1 gene. The sequence may be investigated by using DNA prepared from the peripheral blood of the subject.

The subject of this invention is preferably an Asian, Caucasian, African, African American, or Hispanic.

Other methods of dosing warfarin can be combined with the investigation of the VKORC1 gene. For example, the sequence of the CYP2C9 gene can also be examined based on knowledge available in the art. For example, both CYP2C9*2 and CYP2C9*3 are associated with warfarin sensitivity.

The present invention may be implemented using a kit for determining the dose range of warfarin, comprising at least one component selected from the group consisting of:
(a) a means for detecting sequence A at the - 1639 position of the VKORC1 gene; and
(b) a means for detecting sequence G at the -1639 position of the VKORC1 gene.

The kit can further comprise a means for detecting sequence C and T at the -1639 position of the VKORC1 gene, respectively. Thus, in certain embodiments, the kit may comprise a means for detecting each of the four possible bases at this position. In other embodiments, the kit may contain a means for detecting A, and a means for detecting G, C or T at this position, since the presence of an A at this position is the most indicative of warfarin sensitivity. The means is preferably an oligonucleotide. The kit may optionally comprise the reagents for oligonucleotide hybridization, PCR amplification, and/or instructions of use.

The oligonucleotide, or complement thereof, hybridizes to a region of the VKORC1 gene promoter, wherein the region spans the -1639 position of the promoter and consists of at least 6 nucleotides. Preferably, the oligonucleotide specifically hybridizes with at least 6, 8, 10, 12, 14, 16, 18, 20, 22 or 24 nucleotides spanning the -1639 position of the VKORC1 gene. The oligonucleotide can hybridize to this region wherein the nucleotide at the -1639 position is A, Q C or T, preferably A or G. In certain embodiments, the oligonucleotide may consist of about 15 nucleotides or less, 16-20 nucleotides, 20-25 nucleotides, 25-30 nucleotides, or 30-40 nucleotides. An array comprising the oligonucleotide of this invention is also provided.

The details of one or more embodiments of the invention are set forth in the accompanying drawings and the description below. Other features, objects, and advantages of the invention will be apparent from the description and drawings, and from the claims.

### DESCRIPTION OF DRAWINGS

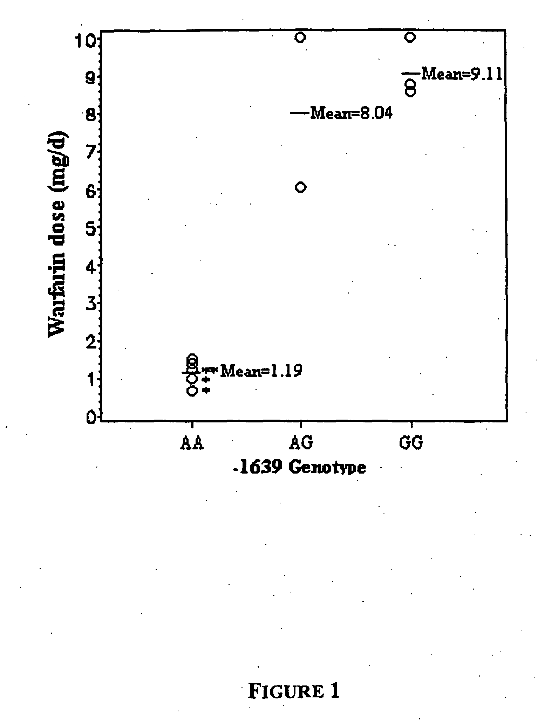

Figure 1. Scatter plot of warfarin doses against the VKORC1 -1639 genotype. Warfarin doses in selected patients with warfarin sensitivity or resistance (see Table 1 for details) were plotted against different genotypes at the VKORC1 promoter -1639 position. *Individuals with CYP2C9 variants including CYP2C9*3, T299A (i.e., amino acid residue 299 changed from T to A) and P382L (i.e., amino acid residue 382 changed from P to L).

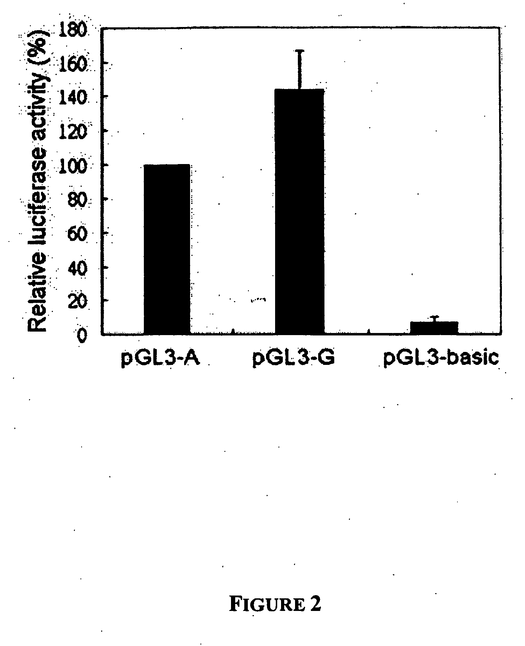

Figure 2. The relative measurements of luciferase activity levels in HepG2 cells. pGL3 luciferase reporter containing either the A (pGL3-A) or G allele (pGL3-G) at the promoter -1639 position. The average of the data from 9 experiments is presented, and the error bars represents standard deviation, pGL3-basic was used as control without any promoter sequence inserted.

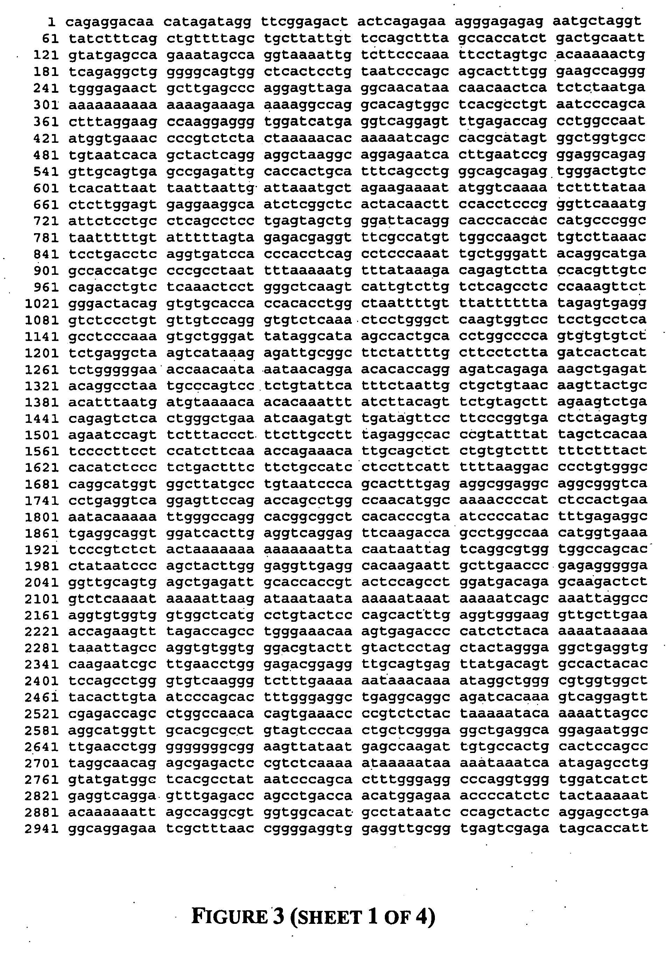

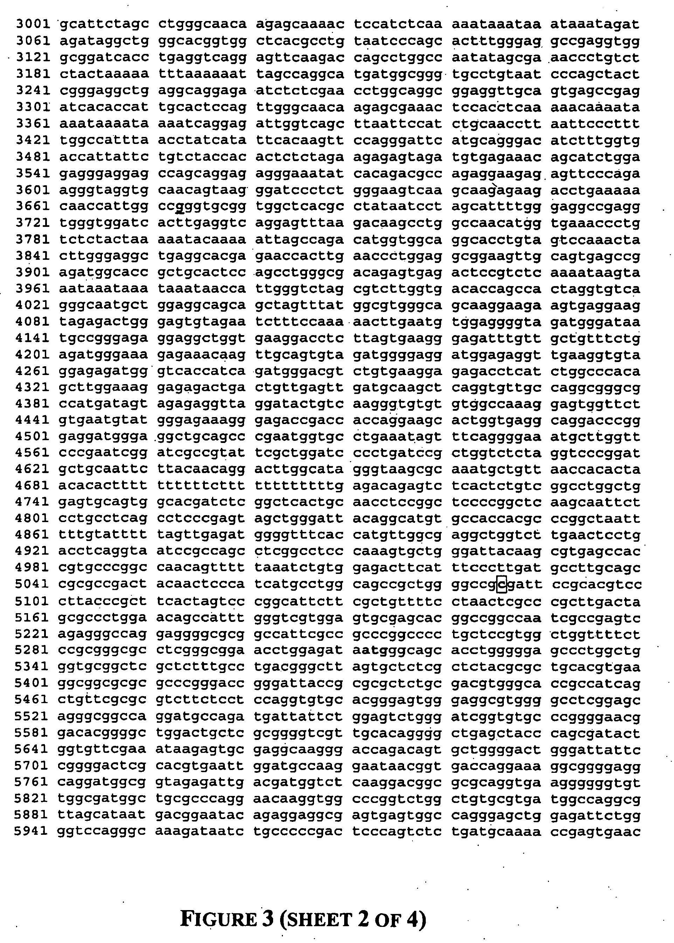

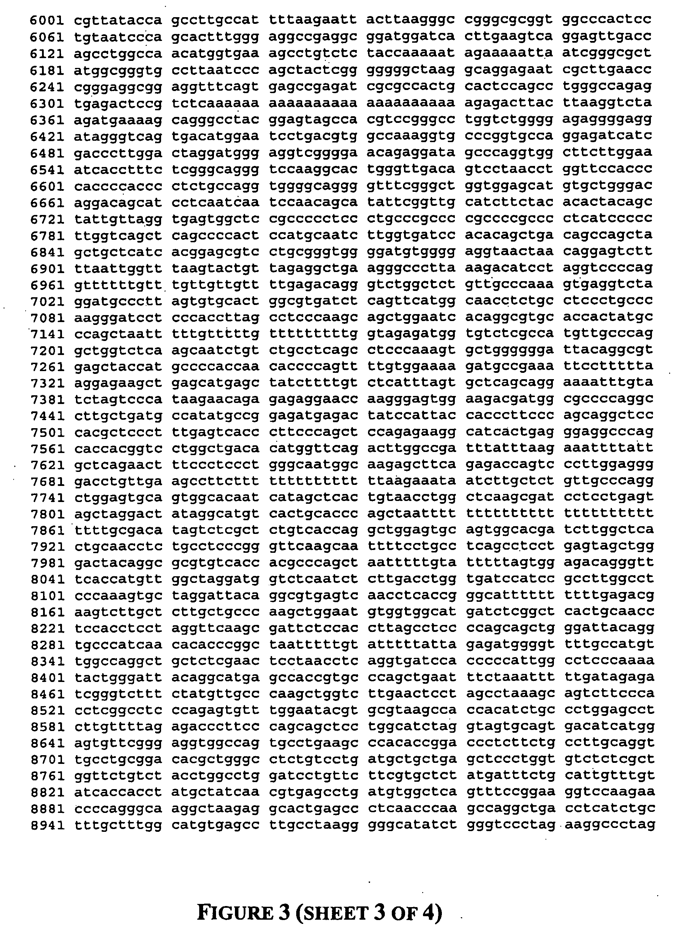

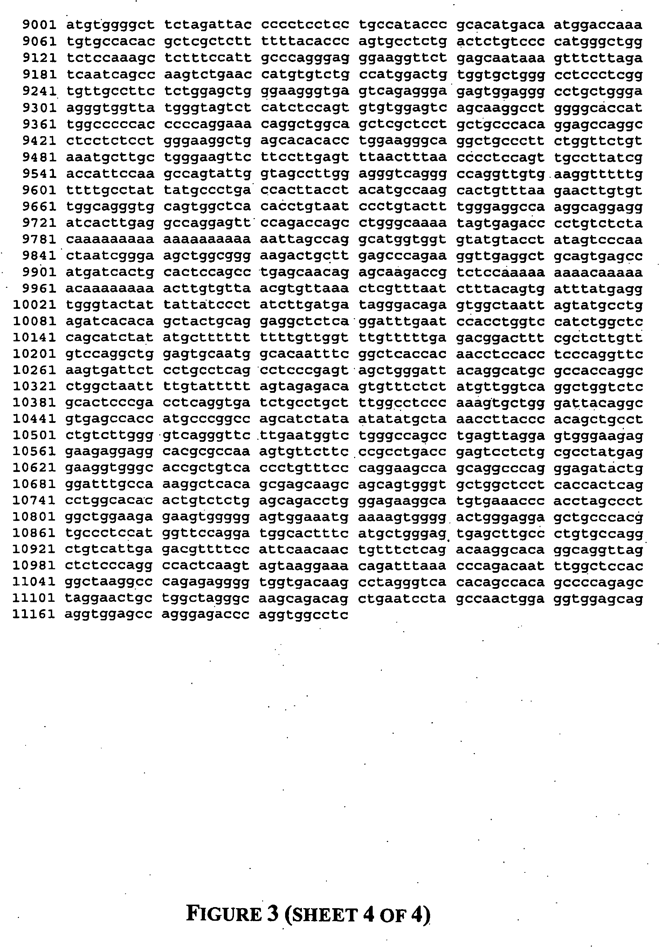

Figure 3. The genomic sequence of the VKORC1 gene (Genbank Accession No. AY587020). The transcription start site is at nucleotide number 5086 (bolded and boxed) in this figure, which is designated as +1 of the gene in the traditional nomenclature. The A of the ATG translation initiation codon (bolded) is at nucleotide number 5312 in this figure, which is recommended by the Human Genome Variation Society to be designated as +1 in the new nomenclature system. The promoter polymorphism described in this invention is underlined and bolded (nucleotide number 3673 in this sequence), which is at -1413 in the traditional system and -1639 in the new, recommended system.

### DETAILED DESCRIPTION

We discovered that a polymorphism in the promoter of the VKORC1 gene is associated with warfarin sensitivity. This polymorphism can explain both the inter-individual and inter-ethnic differences in warfarin dose requirements. Furthermore, the polymorphism is also associated with promoter activity. The VKORC1 promoter having a G at the -1639 position (numbered with respect to the first nucleotide of the initiation codon) is 44% more active than the promoter having an A at the same position. Patients with the -1639A polymorphism are more sensitive to warfarin. Homozygous AA patients at the -1639 position had the lowest dose requirements, heterozygous AG had intermediate dose requirements and the homozygous GG patients had the highest dosage. Thus, the promoter sequence or activity of the VKORC1 gene of a subject can be used to predict how much warfarin should be prescribed for the subject.

Prior to describing the invention in further detail, the terms used in this application are defined as follows unless otherwise indicated.

### Definition

The term "warfarin" encompasses coumarin derivatives having an anticoagulant activity. A preferred embodiment, warfarin, is 4-hydroxy-3-(3-oxo-1-phenylbutyl)-2H-1-benzopyran-2-one (i.e., 3-α-pheneyl-β-acetylethyl-4-hydroxycoumarin). The current commercial product is a racemic mixture of the R-isomer and the S-isomer. The term "warfarin" encompasses the R-isomer, the S-isomer, any racemic mixture, and any salt thereof. Specifically included as warfarin are Coumadin, Marevan, Panwarfin, Prothromadin, Tintorane, Warfilone, Waran, Athrombin-K, warfarin-deanol, Adoisine, warfarin acid, Coumafene, Zoocoumarin, Phenprocoumon, dicumarol, brodifacoum, diphenadione, chlorophacinone, bromadiolone, and acenocoumarol.

An "oligonucleotide", as used herein, is a molecule comprising 2 to about 100 contiguous nucleotides linked via nucleotide bonds. An oligonucleotide may be at least about 10, 20, 30, 40, 50, or 60 nucleotides long. In addition, an oligonucleotide is preferably up to about 200, and more preferably up to about 150, 100, 75, 50, 40, 30, or 20 nucleotides long.

A hybridization "probe" is an oligonucleotide that binds in a base-specific manner to a complementary strand of nucleic acid. Such probes include peptide nucleic acids. Probes can be any length suitable for specific hybridization to the target nucleic acid sequence. The most appropriate length of the probe may vary depending upon the hybridization method in which it is being used; for example, particular lengths may be more appropriate for use in microfabricated arrays, while other lengths may be more suitable for use in classical hybridization methods. Such optimizations are known to the skilled artisan. Suitable probes and primers typically range from about 8 nucleotides to about 100 nucleotides in length. For example, probes and primers can be about 8-20, 10-30, 15-40, 50-80, and preferably 12-20 nucleotides in length. The nucleotide sequence can correspond to the coding sequence of the gene or to the complement of the coding sequence of the gene.

### Methods and Compositions

Upon comparing the CYP2C9 and VKORC1 DNA sequences of Chinese patients with warfarin sensitivity or resistance (Example 1), we found that CYP2C9 DNA sequence variants were present in only 5.4-7.3% of the Han Chinese population. The variants were primarily CYP2C9*3. In addition, two missense mutations identified in this study, including a novel mutation (P382L) not previously reported, were rare and present only in warfarin-sensitive patients. The CYP2C9 variants also showed different prevalence in different ethnic populations. CYP2C9* 1/*2 was present in 20% of the Caucasian population (17); however, it was completely absent in the Chinese population in this study. Therefore, mutations in CYP2C9 can account for only a small percentage of warfarin sensitivity in the Chinese population and cannot explain the inter-ethnical differences in warfarin dose requirements.

On the other hand, we found that changes in the VKORC1 gene can alter warfarin dosage requirements both inter-individually in the Chinese population and inter-ethnically between Chinese and Caucasians. Patients with the -1639 promoter polymorphism AA genotype had lower dose requirements while the AG/GG genotypes had higher dose requirements (Examples 1 and 2). Furthermore, homozygous AA are rare in the Caucasian population, while this genotype makes up the majority of the Chinese population (Example 3). The differences in the genotype frequencies between Caucasians and Chinese, and the correlation ofAA with warfarin sensitivity, are in concordance with what has been observed clinically that the Chinese population requires a lower dose than the Caucasian population.

Accordingly, one aspect of the present invention provides a method of determining the dose range of warfarin for a subject, comprising investigating the sequence of the promoter of the VKORC1 gene of the subject. The promoter sequence preferably contains less than 10%, 8%, 6%, 4%, 3%, 2%, or 1 % variation within 3 kb from the translation initiation site compared to SEQ ID NO: 1. More preferably, the promoter contains less than 20, 15, 12, 10, 8, 6, 4, 3 or 2 single base variations in this 3 kb region. Such a sequence variation, coupled with a change in promoter activity, is indicative of the dose range of warfarin that should be prescribed.

In particular, the sequence at the -1639 position of the VKORC1 gene is investigated. This SNP alone can be used to predict warfarin sensitivity without having to consult any other genotype. Thus, homozygous AA at this position is indicative of warfarin sensitivity, heterozygous AG indicates intermediate sensitivity, and subjects with homozygous GG are relatively resistant. The actual dosage of warfarin that should be given to subjects who are sensitive, intermediately sensitive, or relatively resistant would vary with the warfarin formulation as well as the indication and symptoms of the subject, and can be determined by the physician prescribing the warfarin. For example, our study (Example 1) shows that in a selected patient group with warfarin sensitivity or resistance, the patients with the lowest dose requirements (mean 1.19 mg/day, range 0.71-1.50 mg/day) had the AA genotype, those with the intermediate dose requirements (mean 8.04 mg/day, range 6.07-10 mg/day) had heterozygous AG, and the highest dose requirements (mean 9.11 mg/day, range 8.57-10 mg/day) were associated with the homozygous GG genotype. In randomly selected patients (Example 2), the AA genotype is associated with a lower maintenance dose (2.61 +/- 1.10 my/day) than AG and GG (3.81 +/- 1.24 mg/day). These ranges can serve as the starting points in dosing warfarin.

The genomic sequence of the VKORC1 gene is available as Genbank Accession No. AY587020 (SEQ ID NO:1; Figure 3). The sequence of VKORC1 isoform 1 mRNA is available as Accession Number NM_024006.4. In both of these sequences, the start site of transcription is designated as +1, thus the promoter polymorphism indicative of warfarin sensitivity described herein would be located at the -1413 position. However, according to the recent recommendations by the Human Genome Variation Society (HGVS) for the description of sequence variations (http://www.genomic.unimelb.edu.au/mdi/ mutnomen/), the promoter SNP is recommended to be described in relation to the A of the ATG translation initiation codon. Under this nomenclature, the A of the ATG translation initiation codon (Met) of NM_024006.4 or AY587020 would be position +1, the promoter SNP described herein would be at position -1639, and the G>A polymorphism at this position would be referred to as "NM_024006.4:c.-1639G>A". It should be clarified that the -1639 G>A polymorphism (or more precisely "NM_024006.4:c.-1639G>A") of this disclosure is the same as the -1413 G>A polymorphism according to traditional promoter sequence numbering.

In addition to the specific polymorphism (e.g., AA, AG or GG at the -1639 position), genetic markers that are linked to each of the specific SNPs can be used to predict the corresponding warfarin sensitivity as well. This is because genetic markers near the SNP of interest tend to co-segregate, or show a linkage disequilibrium, with the SNP of interest. Consequently, the presence of these markers (equivalent genetic markers) is indicative of the presence of the SNP of interest, which, in turn, is indicative of the level of warfarin sensitivity.

The VKORC1 -1639 A > G promoter polymorphism is in linkage disequilibrium with the VKORC1 1173 C>T intronic polymorphism recently reported by D'Andrea et al. (27) and may explain their results that Italian patients with the 1173 TT genotype had the lower average daily dose than the CT or CC genotype. Our study also shows that the 3730 (i.e., rs7294) G > A polymorphism, which is located in the 3' untranslated region, is in linkage disequilibrium with -1639 A > G and 1173 C > T in the Chinese population. Specifically, the 3730G allele is associated with the -1639A allele and the 1173T allele. Other equivalent genetic markers of the -1639 SNP include rs9934438 (intron 1), rs8050894 (intron 2) and rs2359612 (intron 2). Thus, -1639A is linked with T at rs9934438, C at rs8050894, T at rs2359612 and G at rs7294, while -1639G is linked with C at rs9934438, G at rs8050894, C at rs2359612 and A at rs7294.

The equivalent genetic marker can be any marker, including microsatellites and single nucleotide polymorphism (SNP) markers. Preferably, the useful genetic markers are about 200 kb from the VKORC1 -1639 position or less. More preferably, the markers are about 100 kb, 80 kb, 60 kb, 40kb, 20 kb, 15 kb, 10 kb, or 5 kb from the VKORC1 -1639 position or less.

One aspect of the present invention provides oligonucleotides that are capable of hybridizing to the SNPs of this invention. The oligonucleotide will be useful, for example, as a hybridization probe or primer for the detection of the promoter sequence of the VKORC1 gene. The oligonucleotide preferably comprises the sequence TGGCCGGGTGC (3668 to 3678 of SEQ ID NO: 1), or the complement thereof. For the purpose of hybridization, it is preferable that the sequence corresponding the -1639 position is near the center of the oligonucleotide. Preferably, there are at least 4 nucleotides on each side of the sequence corresponding to -1639. The -1639 corresponding position is more preferably flanked by at least 5, 6, 7, 8 or 9 nucleotides on each side.

Hybridizations are usually performed under stringent conditions, for instance, at a salt concentration of no more than 1 M and a temperature of at least 25°C. For example, conditions of 5xSSPE (750 mM NaCl, mM NaPhosphate, mM EDT A, pH 7.4) and a temperature of 25-30°C, or equivalent conditions thereof, are suitable for single nucleotide-specific probe hybridizations. More preferably, a low stringent wash after hybridization is conducted, which includes, for example, 42°C, 5xSSC, and 0.1% SDS; or 50°C, 2xSSC, and 0.1% SDS. High-stringent wash conditions are most preferable and include, for example, 65°C, 0.1xSSC, and 0.1% SDS. Equivalent conditions can be determined by varying one or more of the parameters, as known in the art, while maintaining a similar degree of identity or similarity between the target nucleotide sequence and the primer or probe used.

The -1639 promoter SNP is located in an E-Box (the consensus sequence of E-boxes is CANNTG), and within a short distance (200 bp), there are three additional E-boxes. E-boxes have been shown to be important elements for mediating cell/tissue type specific transcription, such as in muscle, neurons, liver and pancreas (28, 29). Changing the second base from A to G as observed at the -1639 site would abolish the E-box consensus and alter the promoter activity. This was clearly demonstrated by the promoter assay that in HepG2 cells the promoter activity was increased by 44% when the consensus sequences was abolished (Figure 2). Without wishing to be limited by theory, this suggests that the E-box may function as a repressor binding site in HepG2. Since HepG2 was derived from a hepatoma, it is likely that the -1639 E-box represses transcription in the liver.

The fact that people with the -1639 GG genotype require a higher warfarin dose can be explained as follows. The VKORC1 gene codes for subunit 1 of vitamin K epoxide reductase complex, which is responsible for regenerating the reduced form of vitamin K. The reduced form of vitamin K is required by gamma-carboxylase, and gamma-carboxylation of vitamin K-dependent clotting factors (factor II, VII, IX, and X) is essential for blood clotting. When VKORC1 promoter activity is increased, an elevated level of VKORC1 mRNA can lead to higher VKOR activity (25) and thus enhance the efficiency of regeneration of reduced vitamin K. Thus, gamma-carboxylation of the vitamin K dependent clotting factors is enhanced due to the higher level of reduced vitamin K. Warfarin acts by blocking clotting factor synthesis, and having more active clotting factors would require more warfarin for its anti-coagulation effect. Since liver is the primary organ for the synthesis of vitamin K dependent clotting factors and VKORC1's expression is highest in the liver, a 44% change in the level of VKORC1 in the liver is likely to have a significant impact on the blood clotting process. Thus, warfarin dose requirements are associated with VKORC1 gene polymorphism and its promoter activity.

We contemplate that any sequence at the -1639 position of the VKORC1 promoter, other than an A, will destroy the E-box and increase promoter activity. An increase in promoter activity, in turn, increases warfarin dose requirements. Thus, the promoter sequence, particularly the sequence at the -1639 position, is indicative of warfarin dosages. Furthermore, other embodiments of the present invention provide methods of dosing warfarin, or determining warfarin sensitivity, by detecting the promoter activity of the VKORC1 gene. Similarly, the level of the gene product of the VKORC1 gene (either mRNA or protein) or VKOR activity can also reflect warfarin dose requirements. A VKORC1 promoter activity, mRNA level, protein level, or VKOR activity that is at least 10%, 15%, 20%, 25%, 30%, 35% or 40% more than that of a subject having the AA genotype is indicative of the requirement for a higher dose of warfarin than the subject with the AA genotype.

Methods of determining promoter activities or levels of mRNA or proteins are well known in the art. In certain embodiments, PCR can be employed to detect mRNA level. In other embodiments, specific antibodies are used to measure the VKORC1 protein. Promoter activities can be examined, for example, by isolating the promoter sequence from the subject of interest using a peripheral blood sample, linking the promoter sequence to a reporter gene, expressing the reporter gene, and determining the amount of the reporter produced. Methods of measuring VKOR activities are also known in the art.

The following examples are offered to illustrate this invention and are not to be construed in any way as limiting the scope of the present invention. While this invention is particularly shown and described with references to preferred embodiments thereof, it will be understood by those skilled in the art that various changes in form and details may be made therein without departing from the spirit and scope of the invention as defined by the appended claims.

### EXAMPLES

In the examples below, the following abbreviations have the following meanings. Abbreviations not defined have their generally accepted meanings.
- °C =: degree Celsius
- hr =: hour
- min =: minute
- sec or s =: second
- µM =: micromolar
- mM =: millimolar
- M =: molar
- ml =: milliliter
- µl =: microliter
- mg =: milligram
- µg =: microgram
- mol =: mole
- pmol =: picomole
- ASO =: allele specific oligonucleotide
- CYP2C9 =: cytochrome P450, subfamily IIC, polypeptide 9
- IND =: international normalized ratio
- LD =: linkage disequilibrium
- NTP =: nucleoside triphosphate
- PBS =: phosphate buffered saline
- PCR =: polymerase chain reaction
- SNP =: single nucleotide polymorphism
- VKORC1 =: vitamin K epoxide reductase complex, subunit 1

### Materials and Methods

### Patients

We recruited 16 patients who received warfarin either at low or high dose from cardiovascular clinics of four major medical centers in Taiwan (National Taiwan University Hospital, Kaohsiung Medical University Hospital, Taipei General Veteran Hospital, and Shin-Kong Wu Ho-Su Memorial Hospital). The mean maintenance dose of warfarin in Chinese patients is 3.3 mg/day (8, 9). Therefore, we considered patients who received maintenance dose ≤ 1.5 mg per day as warfarin sensitive (Table 1, 11 patients) and patients on warfarin maintenance dose ≥ 6 mg per day as warfarin resistant (Table 1, 5 patients). The definition of the warfarin-resistant patients was supported by our own data that none of the randomly selected patients (Table 2) had warfarin dose greater than 6 mg/day. This is in contrast to Caucasian patients, who had an average maintenance dose between 5.1 and 5.5 mg/day (20, 27).

The average daily dose of warfarin was calculated from a one-week-period, and the latest international normalized ratio (INR) of each patient was recorded. The randomly recruited 104 patients who received warfarin, regardless of the dose, had a target INR of 1.4 to 3 from the same 4 hospitals (Table 2). The indications of warfarin were: valve replacement (90 patients), deep vein thrombosis (5 patients), atrial fibrillation (5 patients), and stroke (4 patients). Clinical information (including age, sex, weight, and average daily maintenance dose) was obtained from every participant. At the time of blood draw, every patient had had a constant maintenance dose for at least three weeks. Patients with liver, kidney, gastro-intestinal cancer, or abnormal bleeding problems before warfarin therapy were excluded.

In addition, DNA samples from 92 unrelated Caucasians (Cat. No, HD100CAU, National Institute of General Medical Sciences (NIGMS) Human Genetic Cell Repository, Camden, NJ, USA) were used as Caucasian controls. DNA samples from 95 healthy subjects randomly selected from a biobank under a nationwide population study, in which 3312 Han Chinese descendants were recruited based on the geographic distribution across Taiwan, were used as Chinese controls. The Chinese controls and all participating patients receiving warfarin therapy were unrelated Han Chinese residing in Taiwan. The Han Chinese forms the largest ethnic group in Taiwan, making up roughly 98 percent of the population. None of the participants were aboriginal Taiwanese, which account for the remaining 2% of the Taiwan's population.

The study was approved by the institutional review board, and informed consent was obtained from all of the participants.

### DNA sequencing and single nucleotide polymorphism (SNP) genotyping

Genomic DNA was isolated using the PUREGENE™ DNA purification system (Gentra systems, Minnesota, USA). CYP2C9 and VKORC1 DNA sequence variants were first determined by direct sequencing (Applied Biosystems 3730 DNA analyzer, Applied Biosystems, Foster City, CA, USA) in 16 Han Chinese patients having warfarin sensitivity (≤ 1.5 mg/day, 11 patients) and resistance (≥ 6.0 mg/day, 5 patients). Primers were specifically designed for the intron-exon junctions, exons and 2 kbps upstream of the transcription start site for both CYP2C9 and VKORC1 using the Primer3 PCR primer program (http://fokker.wi.mit.edu/cgi-bin/primer3/primer3_www.cgi). The primers used to detect variants in the VKORC1 promoter were: 5'- CAGAAGGGTAGGTGCAACAGTAA (SEQ ID NO:2; sense strand located 1.5 kb upstream of the transcription start site) and 5'-CACTGCAACTTGTTTCTCTTTCC (SEQ ID NO:3; anti-sense strand located 0.9 kb upstream of the transcription start site). The polymerase chain reactions (PCR) were performed in a final volume of 25 µl, containing 0.4 µM of each primer, 10 mM Tris-HCl (pH8.3), 50 mM KCl, 1.5 mM MgCl₂, 0.2 mM dNTPs and 1 unit *HotStart Taq^{™}* (Qiagen Inc, Valencia, CA, USA). Amplification conditions consisted of an initial denaturation of 12 min at 96°C, followed by 34 PCR cycles in 30 sec at 96°C, 30 sec at 60°C, 40 sec at 72°C.

MALDI-TOF mass spectrometry (SEQUENOM MassARRAY system, Sequenom, San Diego, CA, USA) was then used to screen for the identified variants in 104 randomly selected Chinese patients receiving warfarin, 95 normal Chinese controls and 92 normal Caucasian controls. Briefly, primers and probes were designed using the SpectroDESIGNER software (Sequenom). Multiplex PCR was performed, and unincorporated dNTPs were dephosphorylated using shrimp alkaline phosphatase (Hoffman-LaRoche, Basel, Switzerland), followed by primer extension. The purified primer extension reactions were spotted onto a 384-element silicon chip (SpectroCHIP, Sequenom), analyzed in the Bruker Biflex III MALDI-TOF SpectroREADER mass spectrometer (Sequenom), and the resulting spectrum was processed with SpectroTYPER (Sequenom).

### Luciferase reporter assay

### Plasmid construction

To assay for the VKORC1 promoter activity, the VKORC1 promoter encompassing the -1639 polymorphism (from -1798 to -35) from patients with -1639 AA and -1639 GG genotypes was first cloned into the pGEM-T Easy vector (Promega, Madison, WI, USA) with the forward primer: 5'-ccgctcgagtagatgtgagaaacagcatctgg (SEQ ID NO:4; containing an *Xho*I restriction site) and the reverse primer: 5'-cccaagcttaaaccagccacggagcag (SEQ ID NO:5; containing a *Hind*III restriction site). The fragment containing the VKORC1 promoter was released from the pGEM-T Easy vector by digesting the vector with *Xho*I and *Hind*III restriction enzymes and sub-cloned into the multiple cloning sites *(Xho*I and *Hind*III) of pGL3-basic vector (Promega). The pGL-3 vector contains the cDNA encoded for firefly luciferase, which when fused with a promoter, can be used to analyze the inserted promoter activity upon transfection into mammalian cells. The vector containing the -1639 G/G genotype was designated pGL3-G and the vector containing the -1639 A/A genotype was designated pGL3-A. VKORC1 promoter sequences in both vectors were confirmed by direct sequencing analysis.

### Cell culture and dual luciferase reporter assay

HepG2 cells were grown in Dulbecco's modified Eagle medium (DMEM) and 10% fetal calf serum supplemented with 100 units/mL Penicillin, 100 µg/mL Streptomycin and 2 mM L-Glutamine. Twenty-four hours prior to transfection, 1.5 x 10⁵ cells were seeded in each well of a 12-well plate. On the day of transfection, each well was co-transfected with 1.5 µg of the pGL3 vector and 50 ng of the pRL-TK vector (Promega) using lipofectamine 2000 (Invitrogen Corporation, Carlsbad, CA, USA). The pRL-TK vector encoded the *Renilla* luciferase transcribed by a HSV-TK promoter, which was used as an internal control to normalize firefly luciferase expression. Forty-eight hours after transfection, the cells were lysed in passive lysis buffer (Promega). The cell lysate was added to the luciferase substrate (dual luciferase reporter system, Promega) and the Firefly and *Renilla* luciferase activities were measured with a luminometer (SIRIUS, Pforzheim, Germany).

### Statistical analysis

The genotype frequencies of each SNP were counted. The Chi-square test was used to compare genotype frequencies of each SNP for the three sample groups. T-test and Wilcoxon-Mann-Whitney test were performed for multiple comparisons of mean dose levels among the different genotype groups. Inter-marker linkage disequilibrium was assessed by two measures, D' and r², calculated using Graphical Overview of Linkage Disequilibrium (GOLD,
http://www.sph.umich.edu/csg/abecasis/GOLD/).

### EXAMPLE 1

### CYP2C9 and VKORC1 DNA sequence variants in selected Chinese patients with warfarin sensitivity or resistance

The mean maintenance dose of warfarin in Chinese is 3.3 mg/day (8, 9). Therefore, we considered patients who received maintenance dose ≤ 1.5 mg per day as warfarin sensitive (Table 1, 11 patients) and patients on warfarin maintenance dose ≥ 6 mg per day as warfarin resistant (Table 1, 5 patients, see Materials and Methods for further details). Sequencing of the coding regions, exon-intron junctions, and the promoter region of the CYP2C9 gene revealed three sequence variants in 4 of the 11 warfarin sensitive patients. The variants were: 1075 A > C (1359L known as CYP2C9*3), 895 A > G (T299A), and 1145 C > T (P382L). CYP2C9*3 was detected in 3 patients (subjects 1, 3, and 5, Table 1). Subject 5, in addition to CYP2C9*3, also had the 895 A > G (T299A) change as previously described (7). A novel exonic mutation, 1145 C >T (P382L), was detected in the fourth patient (subject 6). As shown in Table 1, CYP2C9 gene variants were not present in all warfarin sensitive patients and were completely absent in all warfarin resistant patients.

**Table 1. Patient demographics and DNA sequence variants identified.**

| Case No. | Dose (mg/d) | INR | Age (yr) | Sex | Weight (kg) | VKORC1 -1639 | VKORC1 1173 | #CYP2C9 variants |
|---|---|---|---|---|---|---|---|---|
| Warfarin sensitive group (dose ≤ 1.5 mg/day, n=11) | | | | | | | | |
| 1 | 0.71 | 3.23 | 65 | F | 42 | AA | TT | CYP2C9*3 |
| 2 | 1 | 2.69 | 74 | M | 65 | AA | TT | normal |
| 3 | 1 | 3.23 | 70 | M | 66 | AA | TT | CYP2C9*3 |
| 4 | 1.25 | 1.5 | 84 | F | 50 | AA | TT | normal |
| 5 | 1.25 | 1.96 | 68 | M | 75 | AA | TT | CYP2C9*3 895A>G(T299A) |
| 6 | 1.25 | 2.5 | 71 | F | 70 | AA | CT | 1145C>T(P382L) |
| 7 | 1.25 | 2 | 71 | F | 80 | AA | TT | normal |
| 8 | 1.25 | 2.9 | 72 | F | 67 | AA | TT | normal |
| 9 | 1.25 | 1.59 | 67 | M | 58 | AA | TT | normal |
| 10 | 1.43 | 2.05 | 58 | F | 42 | AA | TT | normal |
| 11 | 1.5 | 2.24 | 61 | M | 65 | AA | TT | normal |

| Warfarin resistant group (dose≥ 6mg/day, n=5) | | | | | | | | |
|---|---|---|---|---|---|---|---|---|
| 12 | 6.07 | 2.82 | 48 | F | 52 | AG | CT | normal |
| 13 | 8.57 | 2.09 | 63 | F | 58 | GG | CC | normal |
| 14 | 8.75 | 2.32 | 26 | M | 88 | GG | CC | normal |
| 15 | 10 | 1.3 | 46 | M | 64 | AG | CT | normal |
| 16 | 10 | 2.33 | 58 | F | 61 | GG | CC | normal |

We also sequenced the promoter, coding regions, and the exon-intron junctions of the VKORC1 gene. One variant (3730 G>A) which was located in 3' untranslated region (UTR) of VKORC1 was detected. In addition, a promoter polymorphism, -1639 G > A (or -1413 using transcription start site as +1) was detected in the upstream region of VKORC1. This polymorphism showed an association with warfarin sensitivity in that all warfarin sensitive patients were homozygous AA at position -1639. The warfarin-resistant patients, on the other hand, were either heterozygous AG or homozygous GG (Table 1). When warfarin doses were plotted against the -1639 genotypes, it was demonstrated that patients with the AA genotype had the lowest dose requirements (mean 1.19 mg/day, range 0.71-1.50 mg/day), heterozygous AG had intermediate dose requirements (mean 8.04 mg/day, range 6.07-10 mg/day), and the homozygous GG genotype had the highest dose requirements (mean 9.11 mg/day, range 8.57-10 mg/day) (Figure 1).

In addition to the -1639 G > A, an intron 1 polymorphism 1173 C > T, which has been described by D'Andrea et al. (27), was also identified in our patients. These two polymorphisms appeared to be in strong linkage disequilibrium (LD) (Table 1). Warfarin-sensitive patients with the -1639 AA genotype were found to be associated with the 1173 homozygous TT except for one patient (subject 6). In warfarin-resistant patients, patients with the -1639 heterozygous AG genotype were found to be associated with 1173 heterozygous CT, and homozygous -1639 GG was found to be associated with 1173 homozygous TT (Table 1).

### EXAMPLE 2

### VKORC1 -1639 G>A polymorphism in random Chinese patients receiving warfarin

To further investigate whether the -1639 G > A polymorphism was associated with the inter-individual differences in warfarin dosage, we genotyped 104 patients receiving warfarin regardless of the dose, using MALDI-TOF mass spectrometry. The AA and AG/GG groups did not differ with respect to age, sex, and INR (Table 2). Only two patients were found to be homozygous for GG, and they were grouped together with AG for statistic analysis. We analyzed the data regardless of the presence of other confounding variables, such as diet or other medications. As shown in Table 2, the AA group had significantly lower dose requirements (2.61 mg/day) than the AG/GG group (3.81 mg/day). The differences were significant by either T test (p< 0.0001) or Wilcoxon Mann Whitney test (p = 0.0002) between the AA and AG/GG groups.

**Table 2. Mean doses and other clinical characteristics of randomly selected patients on warfarin stratified according to the genotypes.**

| Genotype | Warfarin dose (mg/day) | INR | Age (Years) | Sex (M/ F) |
|---|---|---|---|---|
| VKORC1-1639 | | | | |
| AA (n=83) | 2.61 ± 1.10^{a} | 2.03 ± 0.45 | 57.5 ± 14.8 | 43/40 |
| AG+GG(n=21) | 3.81 ± 1.24 | 2.08 ± 0.47 | 60.4 ± 13.1 | 13/8 |

| | | | | |
|---|---|---|---|---|
| ^{a}P value of comparison between AA and AG+GG groups. P-value<0.0001 using T test. P-value=0.0002 using Wilcoxon Mann Whitney test. Data represent mean ± SD. | | | | |

### EXAMPLE 3

### Genotype frequencies of VKORC1 -1639 G>A polymorphism and CYP2C9 variants in Chinese and Caucasians

The Chinese population has been known to require a much lower warfarin maintenance dose than the Caucasian population. To test whether differences in the VKORC1 -1639 genotype frequencies could account for the inter-ethnic differences in warfarin dosages, 95 normal Han Chinese subjects and 92 normal Caucasian subjects were genotyped. In the Caucasian population, homozygous AA had the lowest frequency, while the AG and GG genotypes made up the majority of the population (14.2%, 46.7% and 39.1% respectively, Table 3). In the Chinese population, on the contrary, homozygous AA made up the majority of the population (82.1 %), with the rest of the population being heterozygous AG (17.9%). No homozygous GG was detected in this randomly selected population. This ratio was also similar in the 104 warfarin patients in which 79.8% were found to be homozygous AA, 18.3% were heterozygous AG, and the remaining 1.8% were homozygous GG. The differences in genotype frequencies between the Caucasian group and the two Chinese groups were significant, with the p values less than 0.0001. These differences between the two ethic groups (Caucasians and Chinese) and the correlation of AA with warfarin sensitivity are in concordance with the clinical observation that the Chinese population requires a lower dose than the Caucasian population.

**Table 3. Genotype frequencies of VKORC1 promoter polymorphism (-1639 G>A) and CYP2C9 variants in Chinese and Caucasians.**

| Genotype | Caucasian (n=92) Chinese (n=95) | | Random Selected Chinese Warfarin Patients (n=104) |
|---|---|---|---|
| VKORC1 -1639 | | | |
| AA | 13(14.2%) | 78 (82.1%) | 83 (79.8%) |
| AG | 43(46.7%) | 17 (17.9%) | 19 (18.3%) |
| GG | 36(39.1%) | 0 (0%) | 2 (1.9%) |

| CYP2C9 variants^{a} | | | |
|---|---|---|---|
| 2C9*1*2 | 20.4% | 0 (0%) | 0 (0%)^{b} |
| 2C9*2*2 | 0.9% | 0 (0%) | 0 (0%)^{b} |
| 2C9*1*3 | 11.6% | 7 (7.3%) | 4 (5.4%)^{b} |
| 2C9*3*3 | 0.4% | 0 (0%) | 0 (0%)^{b} |

| | | | |
|---|---|---|---|
| P value <0.0001 compared between Caucasian and Chinese population. P value <0.0001 compared between Caucasian and Chinese random selected warfarin patients. P value = 0.817 compared between Chinese and random selected warfarin patients. ^{a}CYP2C9* 1 is wild type of CYP2C9. CYP2C9*2 and CYP2C9*3 are variants with cystine substitutes for arginine at residue 144 and leucine substitutes for isoleucine at residue 359, respectively. Frequencies in Caucasian were obtained from published data (17). ^{b}CYP2C9*3 frequency was derived from genotyping 74 warfarin patients. | | | |

In addition to -1639 G > A, we screened for three intronic polymorphisms (rs9934438, intron 1 1173 C > T; rs8050894, intron 2 g.509+124C; and rs2359612, intron 2 g.509+837C) and one 3' UTR polymorphism (rs7294, 3730 G > A) in the Chinese population. All four polymorphisms were in linkage disequilibrium with the -1639 promoter polymorphism (Inter-marker D' and r2 values = 1.0).

CYP2C9 variants were also genotyped in the Chinese groups. The frequency of CYP2C9*1/*3 was 7.3% in Chinese normal population and 5.4% in the randomly selected Chinese patients receiving warfarin. CYP2C9*2 variant was not detected in the Chinese patients or controls. Compared to the published data on the CYP2C9 variant frequency in Caucasians, the Caucasian population had a much higher frequency of CYP2C9 variants than Chinese (∼30% versus 7%), yet Caucasians are more resistant to warfarin. Other missense mutations detected in the warfarin sensitive patients, 895 A > G (T299A) and 1145 C > T (P382L) (Table 1) were not found in any of the randomly selected patients and controls, suggesting that these were rare mutations.

### EXAMPLE 4

### VKORC1 promoter activity

The differences in warfarin sensitivity between the AA and the GG genotype patients could be explained by changes in the VKORC1 promoter activity. The -1639 promoter SNP was located in the second nucleotide of an E-Box (CANNTG), and therefore, this polymorphism would alter the E-box consensus sequence and could lead to changes in the VKORC1 promoter activity. To test this hypothesis, the VKORC1 promoter encompassing the -1639 position was PCR-amplified from patients with the -1639 AA or GG genotype and cloned into the pGL-3 vector. HepG2 cell (a human hepatoma cell line) was chosen for the promoter assay because VKORC1 is expressed at the highest level in the liver. A total of 9 experiments were performed, and all demonstrated consistent results (shown in Figure 2). The -1639 G VKORC1 promoter exhibited higher luciferase activity (approximately 44% higher) compared to the -1639 A promoter. The pGL-3 basic vector control did not have any promoter inserted in the multiple cloning site and had a negligible amount of luciferase activity. Therefore, the sequence at the -1639 position is important for promoter activity, and higher promoter activity (e.g., -1639G) is associated with higher warfarin dose requirements.

### EXAMPLE 5

### Combination of the VKORC1 -1639 SNP and CYP2C9 variants in warfarin dosing

Although the VKORC1 -1639 SNP alone can be used to predict warfarin sensitivity, this SNP can optionally be combined with other genetic marker(s). For example, the sequences of both the VKORC1 gene and the CYP2C9 gene can be examined in warfarin dosing. The following table illustrates the suggested Coumadin doses for each haplotype when these two genes are both considered:

| VKORC1 -1639 | CYP2C9 variants | Suggested Dose (mg/day) |
|---|---|---|
| GG | *1 | 5 |
| | *2 | 3.75 |
| | *3 | 3.75 |
| AG | *1 | 3.75 |
| | *2 | 2.5 |
| | *3 | 2.5 |
| AA | *1 | 2.5 |
| | *2 | 1.25 |
| | *3 | 1.25 |

## Claims

1. A method of predicting a dose range of a warfarin for a human patient, comprising:
investigating the nucleotide at the 3673 position of SEQ ID NO:1 of the patient;
determining whether the patient is warfarin sensitive or resistant based on the nucleotide at the 3673 position; wherein homozygous AA, heterozygous AG, and homozygous GG at position 3673 indicate that the patient is warfarin sensitive, intermediate sensitive and resistant, respectively; and
predicting a warfarin dosage range based on the warfarin sensitivity or resistance of the patient.

2. The method of claim 1 wherein the sequence is investigated by using an oligonucleotide that specifically hybridizes with the promoter of the VKORC1 gene.

3. The method of claim 2 wherein the oligonucleotide specifically hybridizes with at least 6 nucleotides spanning the 3673 position of SEQ ID NO: 1.

4. The method of claim 1 wherein the sequence is investigated by using DNA prepared from the peripheral blood of the subject.

5. The method of claim 1 wherein the subject is an Asian.

6. The method of claim 1 wherein the subject is a Caucasian.

7. The method of claim 1 wherein the subject is an African, African American, or Hispanic.

8. The method of claim 1 further comprising examining the sequence of the CYP2C9 gene, and predicting a warfarin dose range for the patient based on the nucleotide at position 3673 of SEQ ID NO:1 and the sequence of the CYP2C9 gene.

## Patentansprüche

1. Verfahren zum Vorhersagen eines Dosierbereiches von Warfarin für einen menschlichen Patienten, umfassend:
Untersuchen des Nukleotids an der Position 3673 der Sequenz SEQ ID NO:1 des Patienten;
Bestimmen, ob der Patient warfarinsensitiv oder -resistent ist, basierend auf dem Nukleotid an der Position 3673, wobei homozygotes AA, heterozygotes AG und homozygotes GG an der Position 3673 anzeigt, dass der Patient jeweils warfarinsensitiv, durchschnittlich sensitiv oder resistent ist; und
Vorhersagen eines Warfarindosierbereiches basierend auf der Warfarintensitivität oder -resistenz des Patienten.

2. Das Verfahren gemäß Anspruch 1, wobei die Sequenz **dadurch** untersucht wird, dass ein Oligonucleotid verwendet wird, welches selektiv mit dem Promoter des VKORC1-Gens hybridisiert.

3. Das Verfahren gemäß Anspruch 2, wobei das Oligonukleotid selektiv hybridisiert mit wenigstens sechs Nukleotiden, die die Position 3673 der Sequenz SEQ ID NO:1 überspannen.

4. Das Verfahren gemäß Anspruch 1, wobei die Sequenz **dadurch** untersucht wird, dass DNA verwendet wird, die aus dem peripheren Blut des Subjektes präpariert wurde.

5. Das Verfahren gemäß Anspruch 1, wobei das Subjekt ein Asiate ist.

6. Das Verfahren gemäß Anspruch 1, wobei das Subjekt ein Kaukasier ist.

7. Das Verfahren gemäß Anspruch 1, wobei das Subjekt ein Afrikaner, ein Afroamerikaner oder ein Lateinamerikaner ist.

8. Das Verfahren gemäß Anspruch 1, ferner umfassend die Untersuchung der Sequenz des CYP2C9-Gens, und Vorhersagen eines Warfarin Dosierbereiches für den Patienten, basierend auf dem Nukleotid an der Position 3673 der Sequenz SEQ ID NO:1 und der Sequenz des CYP2C9-Gens.

## Revendications

1. Procédé de prédiction d'une plage de dose d'une warfarine chez un patient humain, comprenant les étapes consistant à :
examiner le nucléotide à la position 3673 de la séquence à numéro d'identification 1 (SEQ ID NO:1) du patient,
déterminer si le patient est sensible ou résistant à la warfarine sur la base du nucléotide à la position 3673, un homozygote AA, un hétérozygote AG et un homozygote GG à la position 3673 indiquant que le patient est sensible, moyennement sensible et résistant à la warfarine, respectivement, et
prédire une plage de dosage de warfarine sur la base de la sensibilité ou de la résistance à la warfarine du patient.

2. Procédé selon la revendication 1, dans lequel la séquence est examinée en utilisant un oligonucléotide qui s'hybride de façon spécifique avec le promoteur du gène VKORC1.

3. Procédé selon la revendication 2, dans lequel l'oligonucléotide s'hybride de façon spécifique avec au moins 6 nucléotides incluant la position 3673 de la séquence à numéro d'identification 1.

4. Procédé selon la revendication 1, dans lequel la séquence est examinée en utilisant un ADN préparé à partir du sang périphérique du sujet.

5. Procédé selon la revendication 1, dans lequel le sujet est un asiatique.

6. Procédé selon la revendication 1, dans lequel le sujet est un caucasien.

7. Procédé selon la revendication 1, dans lequel le sujet est un africain, un afro-américain, ou un hispanique.

8. Procédé selon la revendication 1, comprenant en outre les étapes consistant à examiner la séquence du gène CYP2C9 et à prédire une plage de dose de warfarine pour le patient sur la base du nucléotide à la position 3673 de la séquence à numéro d'identification 1 et de la séquence du gène CYP2C9.


### SEQUENCE LISTING

<110> Academia Sinica
<120> Genetic Variants Predicting Warfarin Sensitivity
<130> JB/070217/JH
<140> EP 05855433.8
   <141> 2005-12-21
<150> US 60/638,837
   <151> 2004-12-21
<150> US 60/679,694
   <151> 2005-05-10
<160> 5
<170> FastSEQ for Windows Version 4.0
<210> 1
   <211> 11190
   <212> DNA
   <213> Homo sapiens
<400> 1
   cagaggacaa catagatagg ttcggagact actcagagaa agggagagag aatgctaggt      60
   tatctttcag ctgttttagc tgcttattgt tccagcttta gccaccatct gactgcaatt     120
   gtatgagcca gaaatagcca ggtaaaattg tcttcccaaa ttcctagtgc acaaaaactg     180
   tcagaggctg ggggcagtgg ctcactcctg taatcccagc agcactttgg gaagccaggg     240
   tgggagaact gcttgagccc aggagttaga ggcaacataa caacaactca tctctaatga     300
   aaaaaaaaaa aaaagaaaga aaaaggccag gcacagtggc tcacgcctgt aatcccagca     360
   ctttaggaag ccaaggaggg tggatcatga ggtcaggagt ttgagaccag cctggccaat     420
   atggtgaaac cccgtctcta ctaaaaacac aaaaatcagc cacgcatagt ggctggtgcc     480
   tgtaatcaca gctactcagg aggctaaggc aggagaatca cttgaatccg ggaggcagag     540
   gttgcagtga gccgagattg caccactgca tttcagcctg ggcagcagag tgggactgtc     600
   tcacattaat taattaattg attaaatgct agaagaaaat atggtcaaaa tcttttataa     660
   ctcttggagt gaggaaggca atctcggctc actacaactt ccacctcccg ggttcaaatg     720
   attctcctgc ctcagcctcc tgagtagctg ggattacagg cacccaccac catgcccggc     780
   taatttttgt atttttagta gagacgaggt ttcgccatgt tggccaagct tgtcttaaac     840
   tcctgacctc aggtgatcca cccacctcag cctcccaaat tgctgggatt acaggcatga     900
   gccaccatgc cccgcctaat tttaaaaatg tttataaaga cagagtctta ccacgttgtc     960
   cagacctgtc tcaaactcct gggctcaagt cattgtcttg tctcagcctc ccaaagttct    1020
   gggactacag gtgtgcacca ccacacctgg ctaattttgt ttatttttta tagagtgagg    1080
   gtctccctgt gttgtccagg gtgtctcaaa ctcctgggct caagtggtcc tcctgcctca    1140
   gcctcccaaa gtgctgggat tataggcata agccactgca cctggcccca gtgtgtgtct    1200
   tctgaggcta agtcataaag agattgcggc ttctattttg cttcctctta gatcactcat    1260
   tctgggggaa accaacaata aataacagga acacaccagg agatcagaga aagctgagat    1320
   acaggcctaa tgcccagtcc tctgtattca tttctaattg ctgctgtaac aagttactgc    1380
   acatttaatg atgtaaaaca acacaaattt atcttacagt tctgtagctt agaagtctga    1440
   cagagtctca ctgggctgaa atcaagatgt tgatagttcc ttcccggtga ctctagagtg    1500
   agaatccagt tctttaccct ttcttgcctt tagaggccac ccgtatttat tagctcacaa    1560
   tccccttcct ccatcttcaa accagaaaca ttgcagctct ctgtgtcttt tttctttact    1620
   cacatctccc tctgactttc ttctgccatc ctccttcatt ttttaaggac ccctgtgggc    1680
   caggcatggt ggcttatgcc tgtaatccca gcactttgag aggcggaggc aggcgggtca    1740
   cctgaggtca ggagttccag accagcctgg ccaacatggc aaaaccccat ctccactgaa    1800
   aatacaaaaa ttgggccagg cacggcggct cacacccgta atccccatac tttgagaggc    1860
   tgaggcaggt ggatcacttg aggtcaggag ttcaagacca gcctggccaa catggtgaaa    1920
   tcccgtctct actaaaaaaa aaaaaaatta caataattag tcaggcgtgg tggccagcac 1980
   ctataatccc agctacttgg gaggttgagg cacaagaatt gcttgaaccc gagaggggga 2040
   ggttgcagtg agctgagatt gcaccaccgt actccagcct ggatgacaga gcaagactct 2100
   gtctcaaaat aaaaattaag ataaataata aaaaataaat aaaaatcagc aaattaggcc 2160
   aggtgtggtg gtggctcatg cctgtactcc cagcactttg aggtgggaag gttgcttgaa 2220
   accagaagtt tagaccagcc tgggaaacaa agtgagaccc catctctaca aaaataaaaa 2280
   taaattagcc aggtgtggtg ggacgtactt gtactcctag ctactaggga ggctgaggtg 2340
   caagaatcgc ttgaacctgg gagacggagg ttgcagtgag ttatgacagt gccactacac 2400
   tccagcctgg gtgtcaaggg tctttgaaaa aataaacaaa ataggctggg cgtggtggct 2460
   tacacttgta atcccagcac tttgggaggc tgaggcaggc agatcacaaa gtcaggagtt 2520
   cgagaccagc ctggccaaca cagtgaaacc ccgtctctac taaaaataca aaaattagcc 2580
   aggcatggtt gcacgcgcct gtagtcccaa ctgctcggga ggctgaggca ggagaatggc 2640
   ttgaacctgg gggggggcgg aagttataat gagccaagat tgtgccactg cactccagcc 2700
   taggcaacag agcgagactc cgtctcaaaa ataaaaataa aaataaatca atagagcctg 2760
   gtatgatggc tcacgcctat aatcccagca ctttgggagg cccaggtggg tggatcatct 2820
   gaggtcagga gtttgagacc agcctgacca acatggagaa accccatctc tactaaaaat 2880
   acaaaaaatt agccaggcgt ggtggcacat gcctataatc ccagctactc aggagcctga 2940
   ggcaggagaa tcgctttaac cggggaggtg gaggttgcgg tgagtcgaga tagcaccatt 3000
   gcattctagc ctgggcaaca agagcaaaac tccatctcaa aaataaataa ataaatagat 3060
   agataggctg ggcacggtgg ctcacgcctg taatcccagc actttgggag gccgaggtgg 3120
   gcggatcacc tgaggtcagg agttcaagac cagcctggcc aatatagcga aaccctgtct 3180
   ctactaaaaa tttaaaaaat tagccaggca tgatggcggg tgcctgtaat cccagctact 3240
   cgggaggctg aggcaggaga atctctcgaa cctggcaggc ggaggttgca gtgagccgag 3300
   atcacaccat tgcactccag ttgggcaaca agagcgaaac tccacctcaa aaacaaaata 3360
   aaataaaata aaatcaggag attggtcagc ttaattccat ctgcaacctt aattcccttt 3420
   tggccattta acctatcata ttcacaagtt ccagggattc atgcagggac atctttggtg 3480
   accattattc tgtctaccac actctctaga agagagtaga tgtgagaaac agcatctgga 3540
   gagggaggag ccagcaggag agggaaatat cacagacgcc agaggaagag agttcccaga 3600
   agggtaggtg caacagtaag ggatccctct gggaagtcaa gcaagagaag acctgaaaaa 3660
   caaccattgg ccgggtgcgg tggctcacgc ctataatcct agcattttgg gaggccgagg 3720
   tgggtggatc acttgaggtc aggagtttaa gacaagcctg gccaacatgg tgaaaccctg 3780
   tctctactaa aaatacaaaa attagccaga catggtggca ggcacctgta gtccaaacta 3840
   cttgggaggc tgaggcacga gaaccacttg aaccctggag gcggaagttg cagtgagccg 3900
   agatggcacc gctgcactcc agcctgggcg acagagtgag actccgtctc aaaataagta 3960
   aataaataaa taaataacca ttgggtctag cgtcttggtg acaccagcca ctaggtgtca 4020
   gggcaatgct ggaggcagca gctagtttat ggcgtgggca gcaaggaaga agtgaggaag 4080
   tagagactgg gagtgtagaa tctttccaaa aacttgaatg tggaggggta gatgggataa 4140
   tgccgggaga ggaggctggt gaaggacctc ttagtgaagg gagatttgtt gctgtttctg 4200
   agatgggaaa gagaaacaag ttgcagtgta gatggggagg atggagaggt tgaaggtgta 4260
   ggagagatgg gtcaccatca gatgggacgt ctgtgaagga gagacctcat ctggcccaca 4320
   gcttggaaag gagagactga ctgttgagtt gatgcaagct caggtgttgc caggcgggcg 4380
   ccatgatagt agagaggtta ggatactgtc aagggtgtgt gtggccaaag gagtggttct 4440
   gtgaatgtat gggagaaagg gagaccgacc accaggaagc actggtgagg caggacccgg 4500
   gaggatggga ggctgcagcc cgaatggtgc ctgaaatagt ttcaggggaa atgcttggtt 4560
   cccgaatcgg atcgccgtat tcgctggatc ccctgatccg ctggtctcta ggtcccggat 4620
   gctgcaattc ttacaacagg acttggcata gggtaagcgc aaatgctgtt aaccacacta 4680
   acacactttt tttttctttt tttttttttg agacagagtc tcactctgtc ggcctggctg 4740
   gagtgcagtg gcacgatctc ggctcactgc aacctccggc tccccggctc aagcaattct 4800
   cctgcctcag cctcccgagt agctgggatt acaggcatgt gccaccacgc ccggctaatt 4860
   tttgtatttt tagttgagat ggggtttcac catgttggcg aggctggtct tgaactcctg 4920
   acctcaggta atccgccagc ctcggcctcc caaagtgctg ggattacaag cgtgagccac 4980
   cgtgcccggc caacagtttt taaatctgtg gagacttcat ttcccttgat gccttgcagc 5040
   cgcgccgact acaactccca tcatgcctgg cagccgctgg ggccgcgatt ccgcacgtcc 5100
   cttacccgct tcactagtcc cggcattctt cgctgttttc ctaactcgcc cgcttgacta 5160
   gcgccctgga acagccattt gggtcgtgga gtgcgagcac ggccggccaa tcgccgagtc 5220
   agagggccag gaggggcgcg gccattcgcc gcccggcccc tgctccgtgg ctggttttct 5280
   ccgcgggcgc ctcgggcgga acctggagat aatgggcagc acctggggga gccctggctg 5340
   ggtgcggctc gctctttgcc tgacgggctt agtgctctcg ctctacgcgc tgcacgtgaa 5400
   ggcggcgcgc gcccgggacc gggattaccg cgcgctctgc gacgtgggca ccgccatcag 5460
   ctgttcgcgc gtcttctcct ccaggtgtgc acgggagtgg gaggcgtggg gcctcggagc 5520
   agggcggcca ggatgccaga tgattattct ggagtctggg atcggtgtgc ccggggaacg 5580
   gacacggggc tggactgctc gcggggtcgt tgcacagggg ctgagctacc cagcgatact 5640
   ggtgttcgaa ataagagtgc gaggcaaggg accagacagt gctggggact gggattattc 5700
   cggggactcg cacgtgaatt ggatgccaag gaataacggt gaccaggaaa ggcggggagg 5760
   caggatggcg gtagagattg acgatggtct caaggacggc gcgcaggtga aggggggtgt 5820
   tggcgatggc tgcgcccagg aacaaggtgg cccggtctgg ctgtgcgtga tggccaggcg 5880
   ttagcataat gacggaatac agaggaggcg agtgagtggc cagggagctg gagattctgg 5940
   ggtccagggc aaagataatc tgcccccgac tcccagtctc tgatgcaaaa ccgagtgaac 6000
   cgttatacca gccttgccat tttaagaatt acttaagggc cgggcgcggt ggcccactcc 6060
   tgtaatccca gcactttggg aggccgaggc ggatggatca cttgaagtca ggagttgacc 6120
   agcctggcca acatggtgaa agcctgtctc taccaaaaat agaaaaatta atcgggcgct 6180
   atggcgggtg ccttaatccc agctactcgg gggggctaag gcaggagaat cgcttgaacc 6240
   cgggaggcgg aggtttcagt gagccgagat cgcgccactg cactccagcc tgggccagag 6300
   tgagactccg tctcaaaaaa aaaaaaaaaa aaaaaaaaaa agagacttac ttaaggtcta 6360
   agatgaaaag cagggcctac ggagtagcca cgtccgggcc tggtctgggg agaggggagg 6420
   atagggtcag tgacatggaa tcctgacgtg gccaaaggtg cccggtgcca ggagatcatc 6480
   gacccttgga ctaggatggg aggtcgggga acagaggata gcccaggtgg cttcttggaa 6540
   atcacctttc tcgggcaggg tccaaggcac tgggttgaca gtcctaacct ggttccaccc 6600
   caccccaccc ctctgccagg tggggcaggg gtttcgggct ggtggagcat gtgctgggac 6660
   aggacagcat cctcaatcaa tccaacagca tattcggttg catcttctac acactacagc 6720
   tattgttagg tgagtggctc cgccccctcc ctgcccgccc cgccccgccc ctcatccccc 6780
   ttggtcagct cagccccact ccatgcaatc ttggtgatcc acacagctga cagccagcta 6840
   gctgctcatc acggagcgtc ctgcgggtgg ggatgtgggg aggtaactaa caggagtctt 6900
   ttaattggtt taagtactgt tagaggctga agggccctta aagacatcct aggtccccag 6960
   gttttttgtt tgttgttgtt ttgagacagg gtctggctct gttgcccaaa gtgaggtcta 7020
   ggatgccctt agtgtgcact ggcgtgatct cagttcatgg caacctctgc ctccctgccc 7080
   aagggatcct cccaccttag cctcccaagc agctggaatc acaggcgtgc accactatgc 7140
   ccagctaatt tttgtttttg tttttttttg gtagagatgg tgtctcgcca tgttgcccag 7200
   gctggtctca agcaatctgt ctgcctcagc ctcccaaagt gctgggggga ttacaggcgt 7260
   gagctaccat gccccaccaa caccccagtt ttgtggaaaa gatgccgaaa ttccttttta 7320
   aggagaagct gagcatgagc tatcttttgt ctcatttagt gctcagcagg aaaatttgta 7380
   tctagtccca taagaacaga gagaggaacc aagggagtgg aagacgatgg cgccccaggc 7440
   cttgctgatg ccatatgccg gagatgagac tatccattac cacccttccc agcaggctcc 7500
   cacgctccct ttgagtcacc cttcccagct ccagagaagg catcactgag ggaggcccag 7560
   caccacggtc ctggctgaca catggttcag acttggccga tttatttaag aaattttatt 7620
   gctcagaact ttccctccct gggcaatggc aagagcttca gagaccagtc ccttggaggg 7680
   gacctgttga agccttcttt tttttttttt ttaagaaata atcttgctct gttgcccagg 7740
   ctggagtgca gtggcacaat catagctcac tgtaacctgg ctcaagcgat cctcctgagt 7800
   agctaggact ataggcatgt cactgcaccc agctaatttt tttttttttt tttttttttt 7860
   ttttgcgaca tagtctcgct ctgtcaccag gctggagtgc agtggcacga tcttggctca 7920
   ctgcaacctc tgcctcccgg gttcaagcaa ttttcctgcc tcagcctcct gagtagctgg 7980
   gactacaggc gcgtgtcacc acgcccagct aatttttgta tttttagtgg agacagggtt 8040
   tcaccatgtt ggctaggatg gtctcaatct cttgacctgg tgatccatcc gccttggcct 8100
   cccaaagtgc taggattaca ggcgtgagtc aacctcaccg ggcatttttt ttttgagacg 8160
   aagtcttgct cttgctgccc aagctggaat gtggtggcat gatctcggct cactgcaacc 8220
   tccacctcct aggttcaagc gattctccac cttagcctcc ccagcagctg ggattacagg 8280
   tgcccatcaa cacacccggc taatttttgt atttttatta gagatggggt tttgccatgt 8340
   tggccaggct gctctcgaac tcctaacctc aggtgatcca cccccattgg cctcccaaaa 8400
   tactgggatt acaggcatga gccaccgtgc ccagctgaat ttctaaattt ttgatagaga 8460
   tcgggtcttt ctatgttgcc caagctggtc ttgaactcct agcctaaagc agtcttccca 8520
   cctcggcctc ccagagtgtt tggaatacgt gcgtaagcca ccacatctgc cctggagcct 8580
   cttgttttag agacccttcc cagcagctcc tggcatctag gtagtgcagt gacatcatgg 8640
   agtgttcggg aggtggccag tgcctgaagc ccacaccgga ccctcttctg ccttgcaggt 8700
   tgcctgcgga cacgctgggc ctctgtcctg atgctgctga gctccctggt gtctctcgct 8760
   ggttctgtct acctggcctg gatcctgttc ttcgtgctct atgatttctg cattgtttgt 8820
   atcaccacct atgctatcaa cgtgagcctg atgtggctca gtttccggaa ggtccaagaa 8880
   ccccagggca aggctaagag gcactgagcc ctcaacccaa gccaggctga cctcatctgc 8940
   tttgctttgg catgtgagcc ttgcctaagg gggcatatct gggtccctag aaggccctag 9000
   atgtggggct tctagattac cccctcctcc tgccataccc gcacatgaca atggaccaaa 9060
   tgtgccacac gctcgctctt ttttacaccc agtgcctctg actctgtccc catgggctgg 9120
   tctccaaagc tctttccatt gcccagggag ggaaggttct gagcaataaa gtttcttaga    9180
   tcaatcagcc aagtctgaac catgtgtctg ccatggactg tggtgctggg cctccctcgg    9240
   tgttgccttc tctggagctg ggaagggtga gtcagaggga gagtggaggg cctgctggga    9300
   agggtggtta tgggtagtct catctccagt gtgtggagtc agcaaggcct ggggcaccat    9360
   tggcccccac ccccaggaaa caggctggca gctcgctcct gctgcccaca ggagccaggc    9420
   ctcctctcct gggaaggctg agcacacacc tggaagggca ggctgccctt ctggttctgt    9480
   aaatgcttgc tgggaagttc ttccttgagt ttaactttaa cccctccagt tgccttatcg    9540
   accattccaa gccagtattg gtagccttgg agggtcaggg ccaggttgtg aaggtttttg    9600
   ttttgcctat tatgccctga ccacttacct acatgccaag cactgtttaa gaacttgtgt    9660
   tggcagggtg cagtggctca cacctgtaat ccctgtactt tgggaggcca aggcaggagg    9720
   atcacttgag gccaggagtt ccagaccagc ctgggcaaaa tagtgagacc cctgtctcta    9780
   caaaaaaaaa aaaaaaaaaa aattagccag gcatggtggt gtatgtacct atagtcccaa    9840
   ctaatcggga agctggcggg aagactgctt gagcccagaa ggttgaggct gcagtgagcc    9900
   atgatcactg cactccagcc tgagcaacag agcaagaccg tctccaaaaa aaaacaaaaa    9960
   acaaaaaaaa acttgtgtta acgtgttaaa ctcgtttaat ctttacagtg atttatgagg   10020
   tgggtactat tattatccct atcttgatga tagggacaga gtggctaatt agtatgcctg   10080
   agatcacaca gctactgcag gaggctctca ggatttgaat ccacctggtc catctggctc   10140
   cagcatctat atgctttttt ttttgttggt ttgtttttga gacggacttt cgctcttgtt   10200
   gtccaggctg gagtgcaatg gcacaatttc ggctcaccac aacctccacc tcccaggttc   10260
   aagtgattct cctgcctcag cctcccgagt agctgggatt acaggcatgc gccaccaggc   10320
   ctggctaatt ttgtattttt agtagagaca gtgtttctct atgttggtca ggctggtctc   10380
   gcactcccga cctcaggtga tctgcctgct ttggcctccc aaagtgctgg gattacaggc   10440
   gtgagccacc atgcccggcc agcatctata atatatgcta aaccttaccc acagctgcct   10500
   ctgtcttggg gtcagggttc ttgaatggtc tgggccagcc tgagttagga gtgggaagag   10560
   gaagaggagg cacgcgccaa agtgttcttc ccgcctgacc gagtcctctg cgcctatgag   10620
   gaaggtgggc accgctgtca ccctgtttcc caggaagcca gcaggcccag ggagatactg   10680
   ggatttgcca aaggctcaca gcgagcaagc agcagtgggt gctggctcct caccactcag   10740
   cctggcacac actgtctctg agcagacctg ggagaaggca tgtgaaaccc acctagccct   10800
   ggctggaaga gaagtggggg agtggaaatg aaaagtgggg actgggagga gctgcccacg   10860
   tgccctccat ggttccagga tggcactttc atgctgggag tgagcttgcc ctgtgccagg   10920
   ctgtcattga gacgttttcc attcaacaac tgtttctcag acaaggcaca ggcaggttag   10980
   ctctcccagg ccactcaagt agtaaggaaa cagatttaaa cccagacaat ttggctccac   11040
   ggctaaggcc cagagagggg tggtgacaag cctagggtca cacagccaca gccccagagc   11100
   taggaactgc tggctagggc aagcagacag ctgaatccta gccaactgga ggtggagcag   11160
   aggtggagcc agggagaccc aggtggcctc                                     11190
<210> 2
   <211> 23
   <212> DNA
   <213> Artificial Sequence
<220>
   <223> primer sequence used to detect the variants in the VKORC1 promoter
<400> 2
   cagaagggta ggtgcaacag taa 23
<210> 3
   <211> 23
   <212> DNA
   <213> Artificial Sequence
<220>
   <223> primer sequence used to detect the variants in the VKORC1 promoter
<400> 3
   cactgcaact tgtttctctt tcc     23
   cactgcaact tgtttctctt tcc 23 <210> 4
   <211> 32
   <212> DNA
   <213> Artificial Sequence
<220>
   <223> forward primer containing an XhoI restriction site
<400> 4
   ccgctcgagt agatgtgaga aacagcatct gg 32
<210> 5
   <211> 27
   <212> DNA
   <213> Artificial Sequence
<220>
   <223> reverse primer containing a HindIII restriction site
<400> 5
   cccaagctta aaccagccac ggagcag 27